# EUROPEAN PATENT APPLICATION

(11) **EP 3 524 232 A1**
(43) Date of publication of application: **14.08.2019**
(21) Application number: 17858389.4
(22) Date of filing: 03.10.2017
(51) Int. Cl.: A61K 9/14, A61K 31/436, A61K 47/10, A61K 47/36, A61K 47/38, A61P 37/06

(54) **METHOD FOR PRODUCING SOLID DISPERSION CONTAINING RAPAMYCIN DERIVATIVE**

(30) Priority: 04.10.2016 JP 2016196120
(71) Applicant: Nippon Kayaku Kabushiki Kaisha, Tokyo 100-0005 (JP)
(72) Inventor: KAWAMURA Dai, Tokyo 115-8588 (JP)
(74) Representative: Godemeyer Blum Lenze Patentanwälte Partnerschaft mbB - werkpatent
(86) International application number: PCT/JP2017/035955
(87) International publication number: WO 2018/066551

(57) **Abstract**

It is an object of the present invention to provide a solid dispersion, which suppresses a reduction in the content of an active ingredient caused by oxidation or decomposition of rapamycin or a derivative thereof, can ensure long-term stability, and has high safety. According to the present invention, there is provided a method for producing a solid dispersion comprising, as an active ingredient, rapamycin or a derivative thereof, wherein the method comprises: a first step of mixing a first solvent that is a protic polar solvent, a second solvent that is an aprotic solvent, a water-soluble polymeric carrier, and rapamycin or a derivative thereof, wherein the ratio of the first solvent is higher than 50% by volume of the total volume of the first solvent and the second solvent; and a second step of removing the solvents.

## Description

### Technical Field

The present invention relates to a solid dispersion, in which the stability of rapamycin or a derivative thereof is improved. The rapamycin or a derivative thereof is extremely unstable against oxygen, light, and water, and thus, is problematic in terms of storage stability. The present invention relates to a technique of enhancing the storage stability of such a compound and optimizing a method for producing a solid dispersion, so as to provide a solid dispersion for pharmaceutical preparations, which is more excellent in terms of long-term stability than solid dispersions obtained by all of the currently reported production methods

### Background Art

It has been known that rapamycin (sirolimus) is a macrolide antibiotic discovered from the metabolite of actinomyces and has an immunosuppressive action. Rapamycin has an action to inhibit a mammalian rapamycin target protein (mammalian target of rapamycin; mTOR) that regulates cell division, cell growth, survival, etc. This mTOR is a main serine-threonine kinase, which regulates the synthesis of proteins by stimulation with growth factors, nutrients, etc., and the mTOR has been known to regulate the growth, proliferation and survival of cells, and angiogenesis. Hence, the mTOR inhibitory action of rapamycin has been focused, and the synthesis of a derivative thereof has been attempted. As a result, everolimus and temsirolimus have been discovered as antitumor agents.

A pharmaceutical preparation used to provide a pharmaceutical product comprising rapamycin or a derivative thereof has been reported. Patent Literature 1 discloses that a solution containing a mixture of rapamycin, hydroxypropylmethyl cellulose, lactose and the like is prepared, the solvent is then distilled away from the solution, and the obtained solid dispersion is then formulated. In addition, Patent Literature 2 discloses a tablet comprising everolimus used as a rapamycin, crospovidone used as a disintegrator, colloidal silicon dioxide, and lactose.

Rapamycin or a derivative thereof has been known to have physical properties by which it is extremely instable to oxidation. Hence, an antioxidant is added to a pharmaceutical preparation comprising, as an active ingredient, rapamycin. For instance, to a rapamycin preparation (registered trademark: Rapalimus) and a temsirolimus preparation (registered trademark: Torisel), tocopherol is added. In addition, for everolimus formulations (registered trademark: Afinitor and Certican), a synthetic antioxidant, dibutylhydroxytoluene (BHT) is used.

Regarding a method of stabilizing a rapamycin derivative, Patent Literature 3 discloses that a mixed solution containing everolimus and BHT as an antioxidant is prepared, and the solvent is then removed, so as to obtain a stabilized everolimus solid.

On the other hand, Patent Literature 4 reports that an ethanol solution of everolimus is added to a water-soluble polymer such as hypromellose, and the mixture is then granulated to prepare a solid dispersion, and the solid dispersion is used for everolimus formulation.

### Prior Art Literatures

### Patent Literatures

Patent Literature 1: JP Patent Publication (Kohyo) No. 11-509223 A (1999)
Patent Literature 2: JP Patent Publication (Kohyo) No. 2005-507897 A
Patent Literature 3: JP Patent Publication (Kohyo) No. 2002-531527 A
Patent Literature 4: International Publication WO2013/022201

### Summary of Invention

### Object to be Solved by the Invention

It is an object of the present invention to provide a method for producing a solid dispersion which is used in a pharmaceutical preparation comprising rapamycin or a derivative thereof, wherein the solid dispersion can suppress a reduction in the content of an active ingredient caused by oxidation or decomposition of the rapamycin or a derivative thereof, and can ensure long-term stability. It is another object of the present invention to provide a pharmaceutical preparation comprising rapamycin or a derivative thereof, which can ensure long-term stability under distribution and storage condition for pharmaceutical product.

The present inventor has found that a solid dispersion produced by a method for producing a solid dispersion, comprising a first step of mixing a first solvent that is a protic polar solvent, a second solvent that is an aprotic solvent, a water-soluble polymeric carrier, and rapamycin or a derivative thereof, wherein the ratio of the first solvent is higher than 50% by volume of the total volume of the first solvent and the second solvent, and a second step of removing the solvents, exhibits the effect of stabilizing the rapamycin or a derivative thereof for a long period of time, thereby completing the present invention. Specifically, the present application has the following inventions [1] to [8] as a gist of the invention.

[1] A method for producing a solid dispersion comprising, as an active ingredient, rapamycin or a derivative thereof, comprising:
   (1) a first step of mixing a first solvent that is a protic polar solvent, a second solvent that is an aprotic solvent, a water-soluble polymeric carrier, and rapamycin or a derivative thereof, wherein the ratio of the first solvent is higher than 50% by volume of the total volume of the first solvent and the second solvent; and
   (2) a second step of removing the solvents.

   To maintain the stability of the rapamycin or a derivative thereof in the solid dispersion, it is important to mix the first solvent, the second solvent, the water-soluble polymeric carrier and the rapamycin or a derivative thereof in the first step.
[2] The method for producing a solid dispersion according to the above [1], wherein the first solvent that is a protic polar solvent is one or more types selected from the group consisting of water, C1-C5 alcohol, and C1 and C2 carboxylic acid.
[3] The method for producing a solid dispersion according to the above [1] or [2], wherein the second solvent that is an aprotic solvent is one or more types selected from the group consisting of acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methyl pyrrolidone, 1,3-dimethyl-2-imidazolidinone, dimethyl sulfoxide, dichloromethane, and chloroform.
[4] The method for producing a solid dispersion according to any one of the above [1] to [3], wherein the volume of the first solvent is more than 2 parts by volume and less than 50 parts by volume, with respect to 1 part by volume of the second solvent.
[5] The method for producing a solid dispersion according to any one of the above [1] to [4], wherein the first solvent that is a protic polar solvent is ethanol, and the second solvent that is an aprotic solvent is acetone.
[6] The method for producing a solid dispersion according to any one of the above [1] to [5], wherein, in the first step, the first solvent is used in an amount of 15 parts by volume or more, with respect to 1 part by mass of the water-soluble polymeric carrier.
[7] The method for producing a solid dispersion according to any one of the above [1] to [6], wherein the water-soluble polymeric carrier is a water-soluble synthetic polymer derivative carrier or a water-soluble cellulose derivative carrier.
[8] The method for producing a solid dispersion according to any one of the above [1] to [7], wherein, in the first step, a mixed solution obtained by mixing the water-soluble polymeric carrier with the first solvent and/or the second solvent is mixed with a solution obtained by dissolving the rapamycin or a derivative thereof in the first solvent and/or the second solvent.
[9] The method for producing a solid dispersion according to any one of the above [1] to [8], wherein, in the first step, a stabilizer is added.
[10] The method for producing a solid dispersion according to the above [9], wherein the stabilizer is tocopherol.
[11] The method for producing a solid dispersion according to the above [9] or [10], wherein, in the first step, a mixed solution obtained by mixing the water-soluble polymeric carrier with the first solvent and/or the second solvent, a solution obtained by dissolving the stabilizer in the first solvent and/or the second solvent, and a solution obtained by dissolving the rapamycin or a derivative thereof in the first solvent and/or the second solvent are mixed with one another.
[12] The method for producing a solid dispersion according to any one of the above [1] to [11], wherein, in the first step, sugars are added.
[13] A pharmaceutical preparation comprising a solid dispersion produced by the production method according to any one of the above [1] to [12].

### Advantageous Effects of Invention

According to the present invention, a reduction in the content of an active ingredient caused by oxidation or decomposition of rapamycin or a derivative thereof is suppressed, so that a solid dispersion containing rapamycin or a derivative thereof, which can ensure long-term stability, can be produced. In addition, a pharmaceutical preparation containing rapamycin or a derivative thereof, which can ensure long-term stability as a pharmaceutical product under distribution and storage conditions, can be provided.

### Brief Description of Drawings

Figure 1 shows the results obtained by plotting, over time, the amount of everolimus remaining in a solid dispersion produced by the production method of the present invention, which is stored in a desiccator in which the humidity is controlled at 40°C/saturated saline (relative humidity: approximately 75%) under light-shielding conditions.
Figure 2 shows the results obtained by plotting, over time, the amount of everolimus in a solution, which is prepared by adding each mixed solvent to everolimus to a concentration of 1 mg/mL relative to everolimus, and which is then stored at 25°C.
Figure 3 shows the results obtained by plotting, over time, the amount of everolimus remaining in a mixture, which is prepared by adding everolimus into a mixed solution containing ethanol : acetone = 9 : 1, and which is then stored at 25°C.
Figure 4 shows the results obtained by plotting, over time, the amount of everolimus remaining in a mixture, which is prepared by adding everolimus into ethanol, and which is then stored at 25°C.

### Description of Embodiments

The present invention relates to a method for producing a solid dispersion comprising, as an active ingredient, rapamycin or a derivative thereof, wherein the method comprises: a first step of mixing a first solvent that is a protic polar solvent, a second solvent that is an aprotic solvent, a water-soluble polymeric carrier, and rapamycin or a derivative thereof, wherein the ratio of the first solvent is higher than 50% by volume of the total volume of the first solvent and the second solvent; and a second step of removing the solvents. The details thereof will be described below.

The present invention comprises, as an active ingredient, rapamycin or a derivative thereof.

Rapamycin (common name: Sirolimus) is a compound having a macrolide skeleton that has been isolated from the metabolite of actinomyces, *Streptomyces Hygroscopicus,* separated from the soil of Easter Island.

The term "rapamycin derivative" means a substance prepared by chemically modifying rapamycin used as a mother core. Examples of the rapamycin derivative include 16-O-substituted rapamycin (see, for example, WO 94/022136), 40-O-substituted rapamycin (see, for example, US 5258389 and WO 94/09010), carboxylic acid ester-substituted rapamycin (see, for example, WO 92/05179), amide-substituted rapamycin (see, for example, US 5118677), fluorine-substituted rapamycin (see, for example, US 5100883), and acetal-substituted rapamycin (see, for example, US 5151413). The rapamycin or a derivative thereof of the present invention is not limited thereto, but the aforementioned rapamycin derivatives can be used as applicable preferred compounds.

The rapamycin derivative is preferably a 40-O-substituted rapamycin derivative, in which the hydroxyl group at position 40 of the cyclohexyl group of rapamycin is substituted with a hydroxyalkyl group, a hydroxyalkoxyalkyl group, an acylaminoalkyl group, an aminoalkyl group, or a hydroxy-substituted acyl group.

The rapamycin derivative is more preferably 40-O-(2-hydroxyethyl)rapamycin (everolimus), or 40-O-[3-hydroxy-2-(hydroxymethyl)-2-methylpropanoate]rapamycin (temsirolimus).

As (A) rapamycin or a derivative thereof of the present invention, rapamycin (Sirolimus), everolimus, or temsirolimus is preferably used.

As such (A) rapamycin or a derivative thereof, a compound having a quality that can be sufficiently used as a pharmaceutical product is preferably used.

The solid dispersion produced by the production method of the present invention is a solid, in which rapamycin or a derivative thereof is dispersed in the after-mentioned water-soluble polymeric carrier. In this solid dispersion, other pharmaceutical additives may also be comprised.

The method for producing a solid dispersion of the present invention is characterized in that it comprises a first step of mixing a first solvent that is a protic polar solvent, a second solvent that is other than the protic polar solvent, a water-soluble polymeric carrier, and rapamycin or a derivative thereof, wherein the ratio of the first solvent is higher than the ratio of the second solvent by 50% by volume or more.

Examples of the above-described first solvent that is a protic polar solvent include water, C1-C5 alcohol, and C1 and C2 carboxylic acid. Specific examples include water, methanol, ethanol, 1-propanol, 2-propanol, 1-butanol, 2-butanol, 2-methyl-1-propanol, 3-methyl-1-butanol, 1-pentanol, ethylene glycol, glycerin, formic acid, and acetic acid. Among others, water and C1-C5 alcohol, for example, water, methanol, ethanol, 1-propanol, 2-propanol, 1-butanol, 2-butanol, 2-methyl-1-propanol, 3-methyl-1-butanol, 1-pentanol, ethylene glycol, glycerin and the like are preferably used.

These solvents may be used alone, or may also be a mixed solvent comprising two or more types of solvents. The present invention is not limited to the use of the above-described solvents, but the above-described solvents may be applicable, preferred solvents.

Taking into consideration the subsequent removal of the above-described first solvent, a solvent having a boiling point of 120°C or lower, which can be distilled away under moderate conditions, is preferably used as such a first solvent. Preferred examples of the first solvent include water, methanol, ethanol, 1-propanol, and 2-propanol. Among others, methanol, ethanol, 1-propanol, and 2-propanol are particularly preferably used. These solvents may be used each alone, or may also be used in combination.

Examples of the above-described second solvent that is an aprotic solvent include acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methyl pyrrolidone, 1,3-dimethyl-2-imidazolidinone, dimethyl sulfoxide, dichloromethane, and chloroform.

These solvents may be used alone, or may also be a mixed solvent comprising two or more types of solvents. The present invention is not limited to the use of the above-described solvents, but the above-described solvents may be applicable, preferred solvents.

Taking into consideration the subsequent removal of the above-described second solvent, a solvent having a boiling point of 120°C or lower, which can be distilled away under moderate conditions, is preferably used as such a second solvent. Preferred examples of the second solvent include acetonitrile, acetone, methyl ethyl ketone, methyl acetate, ethyl acetate, isopropyl acetate, isobutyl acetate, tetrahydrofuran, 1,4-dioxane, pentane, heptane, diethyl ether, and t-butyl methyl ether. Among others, acetone, isopropyl acetate and ethyl acetate are particularly preferably used. These solvents may be used each alone, or may also be used in combination.

The above-described first solvent and the above-described second solvent can be preferably uniformly mixed with each other. The combination of the first solvent and the second solvent is not particularly limited, but the combination of the first solvent that is ethanol and the second solvent that is acetone is significantly preferable.

The ratio of the first solvent in the present invention is desirably higher than 50% by volume of the total volume of the first solvent and the second solvent. When the ratio of the first solvent is 100% by volume, the stability of rapamycin or a derivative thereof is reduced. Thus, the ratio of the first solvent is more preferably 60% by volume or more and lower than 100% by volume, and further preferably 65% by volume or more and 95% by volume or less, of the total volume of the first solvent and the second solvent. The ratio of the first solvent may also be 70% by volume or more and 95% by volume or less, or 75% by volume or more and 95% by volume or less, or 80% by volume or more and 95% by volume or less, or 85% by volume or more and 95% by volume or less, of the total volume of the first solvent and the second solvent. The ratio of the first solvent may also be, for example, 70% by volume, 80% by volume, or 90% by volume of the total volume of the first solvent and the second solvent.

The amount of the first solvent used is not particularly limited, and the used amount can be adjusted, as appropriate. The first solvent is used in an amount of preferably 15 parts by volume or more, and more preferably 18 parts by volume or more, with respect to 1 part by mass of the after-mentioned water-soluble polymeric carrier.

The volume of the first solvent is preferably 2 parts by volume or more and 50 parts by volume or less, and more preferably 2 parts by volume or more and 10 parts by volume or less, with respect to 1 part by volume of the above-described second solvent.

The above-described water-soluble polymeric carrier is not particularly limited, as long as it is a water-soluble polymeric carrier that can be applied as an additive for pharmaceutical products. The eluting properties of rapamycin or a derivative thereof comprised as an active ingredient in the solid dispersion depend on the dissolution speed of the polymeric carrier. In the case of using a fat-soluble polymeric carrier, when the fat-soluble polymeric carrier is administered into a living body, the solubility of the fat-soluble polymeric carrier is poor, and thus, the eluting properties of rapamycin or a derivative thereof are considered to be decreased. On the other hand, in the case of using a water-soluble polymer, when the water-soluble polymer is administered into a living body, the water-soluble polymeric carrier is promptly dissolved, and thus, the eluting properties of rapamycin or a derivative thereof in water are ensured. Prompt elution of medicinal components is important for the expression of the effects of the medicinal components. Hence, the water-soluble polymeric carrier is preferably used.

It is considered that the rapamycin or a derivative thereof interacts with the water-soluble polymeric derivative via a hydrogen bond. It is considered that, thereby, the structure of the rapamycin or a derivative thereof is stabilized and exhibits higher stability as compared with a case of other carriers.

Examples of the water-soluble polymeric carrier include a water-soluble synthetic polymer derivative carrier and a water-soluble cellulose derivative carrier. Since the interaction of the rapamycin or a derivative thereof with the water-soluble polymeric carrier via a hydrogen bond is considered to be important for maintaining stability, the water-soluble synthetic polymer derivative carrier and the water-soluble cellulose derivative carrier preferably have a functional group capable of hydrogen-bonding with hydrogen of a hydroxyl group of the rapamycin or a derivative thereof, such as a hydroxyl group, a carboxy group, an amide group, an ether group, an ester group, a carbonyl group, or an amino group.

Examples of the water-soluble synthetic polymer derivative carrier include povidone, copolyvidone, macrogol, polyvinyl alcohol, a methacrylic acid copolymer, and a polyvinylcaprolactam-polyvinyl acetate-polyethylene glycol graft copolymer. These water-soluble synthetic polymer derivative carriers may be used each alone, or may also be used in combination.

As a water-soluble polymeric carrier used in the present invention, a water-soluble cellulose derivative is preferably used. The water-soluble cellulose derivative is a water-soluble polymer, in which some hydrogen atoms of the hydroxyl group of cellulose are substituted with substituents such as methyl groups, ethyl groups, propyl groups, hydroxypropyl groups, or carboxymethyl groups.

The water-soluble cellulose derivative used in the present invention is preferably a water-soluble cellulose derivative that is acceptable as an additive for pharmaceutical products. Specific examples include hydroxypropylmethyl cellulose (hypromellose), hydroxypropyl cellulose, methyl cellulose, ethyl cellulose, hydroxyethyl cellulose, hydroxymethylethyl cellulose, cellacefate, hydroxypropylmethyl cellulose phthalate, hypromellose acetate succinate, hypromellose phthalic ester, carboxymethyl cellulose, and their alkaline metal salts such as sodium salts or potassium salts. Preferred examples include hydroxypropylmethyl cellulose (hypromellose), hydroxypropyl cellulose, methyl cellulose, ethyl cellulose, hypromellose phthalic ester, and hypromellose succinic ester. The water-soluble cellulose derivative is most preferably hydroxypropylmethyl cellulose (hypromellose).

The additive amount of the water-soluble polymeric carrier that may be used in the present invention is preferably 0.5 parts by mass or more with respect to 1 part by mass of the rapamycin or a derivative thereof. It may be more preferably 1 part by mass or more. That is to say, the water-soluble polymeric carrier can be used, as appropriate, in an amount in which the stability of the rapamycin or a derivative thereof is not impaired. The additive amount of the water-soluble polymeric carrier used herein is preferably 0.5 to 50 parts by mass with respect to 1 part by mass of the rapamycin or a derivative thereof. The additive amount of the water-soluble polymeric carrier is more preferably 0.5 to 30 parts by mass, and further preferably 1 to 20 parts by mass.

Besides, the water-soluble polymeric carrier may be in a state in which it is mixed with the first solvent and the second solvent and it is completely dissolved in the solvents, or may also be in a state in which the water-soluble polymeric carrier is suspended in the solvents. Thus, the water-soluble polymeric carrier may be used in any aspects.

The rapamycin or a derivative thereof may be directly added in the form of powders, or may be dissolved in a solvent and may be then added in the form of a solution. The solvent, in which the rapamycin or a derivative thereof is to be dissolved, is not particularly limited, as long as the rapamycin or a derivative thereof is dissolved therein. As such a solvent, it is preferable to use the first solvent and the second solvent applied in the first step. The ratio of the first solvent is desirably higher than 50% by volume. The ratio of the first solvent is more preferably higher than 65% by volume and lower than 100% by volume. Moreover, either the first solvent or the second solvent may be used. When the rapamycin or a derivative thereof is dissolved in either the first solvent or the second solvent, the final ratio of the first solvent in the mixture prepared in the first step is adjusted, such that it preferably becomes higher than 50% by volume, and more preferably becomes higher than 65% by volume and lower than 100% by volume, with respect to the volume of the second solvent. If the first solvent is used alone, the stability of rapamycin or a derivative thereof is considered to be decrease. Accordingly, when the rapamycin or a derivative thereof is dissolved in either the first solvent or the second solvent, it is preferable to use only the second solvent.

The total amount of the first solvent and the second solvent used can be adjusted, as appropriate, such that the rapamycin or a derivative thereof may be completely dissolved in the first solvent and the second solvent. It is preferable to prepare a solution containing the rapamycin or a derivative thereof in a concentration of 0.1 to 1000 mg/mL or less, and the concentration of the rapamycin or a derivative thereof is preferably 0.5 to 500 mg/mL.

Moreover, upon preparation of the solution, the solution may be heated, as appropriate, so that the dissolution of the rapamycin or a derivative thereof may be promoted. The temperature of the solution upon preparation thereof is not particularly limited, but taking into consideration the stability of the rapamycin or a derivative thereof, it is preferable to prepare the solution at 0°C to 80°C.

The order of mixing the first solvent, the second solvent, the water-soluble polymeric carrier, and the rapamycin or a derivative thereof is not particularly limited. After the first solvent, the second solvent and the water-soluble polymeric carrier have been mixed with one another, the rapamycin or a derivative thereof may be mixed therein. Otherwise, after the first solvent, the second solvent and the rapamycin or a derivative thereof have been mixed with one another, the water-soluble polymeric carrier may be mixed therein. Otherwise, after the first solvent, the water-soluble polymeric carrier and the rapamycin or a derivative thereof have been mixed with one another, the second solvent may be mixed therein. Otherwise, after the second solvent, the water-soluble polymeric carrier and the rapamycin or a derivative thereof have been mixed with one another, the first solvent may be added therein. It is preferable that after the first solvent, the second solvent and the water-soluble polymeric carrier have been mixed with one another, the rapamycin or a derivative thereof may be mixed therein. Alternatively, in the case of the first solvent and the second solvent, the total amount of each solvent used may be divided before mixing. That is, a portion of the total amount of the first solvent and the second solvent used in the first step is mixed with either the water-soluble polymeric carrier or the rapamycin or a derivative thereof, and the residual amount thereof is mixed with the other of the water-soluble polymer or the rapamycin or a derivative thereof, and thereafter, the obtained two mixtures may be mixed with each other.

In the above-described first step, it is preferable that, after the first solvent and/or the second solvent have been mixed with the water-soluble polymeric carrier, and the rapamycin or a derivative thereof is mixed with the mixture. It is considered that the water-soluble polymeric carrier interacts with the rapamycin or a derivative thereof via a hydrogen bond, so that the structure of the rapamycin or a derivative thereof is stabilized and thus exhibits high stability. As such, it is considered that, by previously mixing the water-soluble polymeric carrier with a mixed solvent of the first solvent and the second solvent, in which the first solvent that is a protic polar solvent is contained in an amount of higher than 50% by volume with respect to the volume of the second solvent, the hydrogen bond between the water-soluble polymeric carriers can be weakened, and then, by adding the rapamycin or a derivative thereof thereto, the interaction of the water-soluble polymeric carrier with the rapamycin or a derivative thereof can be facilitated.

In the above-described first step, it is preferable that, after the first solvent and/or the second solvent have been mixed with the water-soluble polymeric carrier, the rapamycin or a derivative thereof dissolved in the first solvent and/or the second solvent is mixed with the obtained mixture. It is considered that the water-soluble polymeric carrier interacts with the rapamycin or a derivative thereof via a hydrogen bond, so that the structure of the rapamycin or a derivative thereof is stabilized and thus exhibits high stability. As such, it is considered that, by previously mixing the water-soluble polymeric carrier and the rapamycin or a derivative thereof with the first solvent and/or the second solvent, and then by mixing the obtained mixtures or solutions with each other, the components are easily blended with one another, and the interaction of the water-soluble polymeric carrier with the rapamycin or a derivative thereof can be facilitated.

The method for producing a solid dispersion of the present invention comprises a second step of removing the solvents. As a method of removing the solvents, for example, the solvents can be removed by heating the above-described mixed solution. At that time, it is preferable to apply reduced pressure conditions, or to spray the mixed solution as fine liquid droplets into the gas, so that the solvents are removed under moderate temperature conditions. Alternatively, a solid dispersion can also be obtained by ventilation drying, in which inert gas such as air or nitrogen is supplied into a dryer for drying.

In the method for producing a solid dispersion of the present invention, a stabilizer may be used in the above-described first step.

The stabilizer is not particularly limited, as long as it suppresses the oxidation, photo-degradation or hydrolysis of rapamycin or a derivative thereof and maintains the stability thereof, when it is added to the rapamycin or a derivative thereof. As such stabilizers, known antioxidants or stabilizers exhibiting the effect of stabilizing rapamycin and a derivative thereof can be used.

Examples of the stabilizer include nitrous acid, ascorbic acid, sodium ascorbate, ascorbyl stearate, ascorbyl palmitate, sulfurous acid, alpha-thioglycerol, edetic acid, erythorbic acid, cysteine hydrochloride, citric acid, sodium citrate, disodium hydrogen citrate, sodium dihydrogen citrate, dichloroisocyanuric acid, dibutylhydroxytoluene (BHT), thioglycolic acid, thiomalic acid, tocopherol, trehalose, pyrosulfurous acid, butylhydroxyanisole, 1,3-butylene glycol, pentaerythritol tetrakis[3-(3,5-di-tert-butyl-4-hydroxyphenyl)propionate]benzotriazole, isopropyl gallate, 2-mercaptobenzimidazole, and lecithin. Examples of the stabilizer are not limited to the aforementioned compounds, but these compounds can be preferably applied as stabilizers in the present invention. The above-described stabilizers may be used each alone, or may also be used in combination.

More preferred examples of the stabilizer include sodium ascorbate, ascorbyl stearate, disodium hydrogen citrate, sodium dihydrogen citrate, dibutylhydroxytoluene (BHT), tocopherol, trehalose, butylhydroxyanisole, isopropyl gallate, and lecithin. Most preferred examples of the stabilizer include sodium ascorbate, ascorbyl stearate, disodium hydrogen citrate, dibutylhydroxytoluene (BHT), tocopherol, trehalose, and lecithin.

The above-described stabilizer can be used, as appropriate, in an amount that does not impair the stability of the rapamycin or a derivative thereof. The additive amount of the stabilizer is preferably 0.0001 to 10 parts by mass with respect to 1 part by mass of the rapamycin or a derivative thereof. The additive amount of the stabilizer is more preferably 0.0005 to 1.0 parts by mass, and further preferably 0.001 to 1.0 parts by mass.

Besides, the above-described stabilizer is preferably applied in a state in which it is completely dissolved in a solvent. The stabilizer may be dissolved in a solvent other than the above-described first solvent and the above-described second solvent, and may be then used. The solvent, in which the stabilizer is to be dissolved, is not particularly limited, as long as the stabilizer can be dissolved therein. Examples of the solvent used herein include water, methanol, ethanol, 1-propanol, 2-propanol, 1-butanol, 2-butanol, 2-methyl-1-propanol, 3-methyl-1-butanol, 1-pentanol, ethylene glycol, glycerin, formic acid, acetic acid, acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methyl pyrrolidone, 1,3-dimethyl-2-imidazolidinone, dimethyl sulfoxide, dichloromethane, and chloroform.

These solvents may be used alone, or may also be a mixed solvent comprising two or more types of solvents. The present invention is not limited to the use of the above-described solvents, but the above-described solvents may be applicable, preferred solvents.

In the solid dispersion produced by the production method of the present invention, sugars may be used as a carrier, and such sugars may be added in the first step. Examples of such a carrier include monosaccharides and oligosaccharides. Sugar alcohol and disaccharides are preferable, and one or more types of sugars selected from the group consisting of mannitol, lactose, trehalose, and maltose are preferable. These sugars may be used each alone, or two or more types of sugars may be used in combination. The use of lactose is particularly preferable.

With regard to the additive amount of sugars used in the present invention, sugars may be used in an amount of 0.1 part by mass or more, with respect to 1 part by mass of the rapamycin or a derivative thereof. Sugars may be used in an amount of preferably 0.5 parts by mass or more, and more preferably 1 part by mass or more. It is sufficient, if the sugars are used in an amount of 5 parts by mass or more. In the present invention, since the sugars having a critical relative humidity at 25°C of 95% or more do not particularly cause safety hazard, the upper limit of the use amount thereof is not particularly limited. The use amount of the sugars should be determined within an amount range that is practically usable as a pharmaceutical product.

Taking into consideration the ensuring of the stability of the rapamycin or a derivative thereof and the practically usable amount of pharmaceutical product additives, the sugars are preferably used in an amount of 0.1 to 100 parts by mass, with respect to 1 part by mass of the rapamycin or a derivative thereof. The amount of the sugars is preferably 0.1 to 50 parts by mass, more preferably 0.5 to 50 parts by mass, and further preferably 1 to 50 parts by mass.

In the production method of the present invention, the sugars are preferably added, after the water-soluble polymeric carrier has been mixed with the rapamycin or a derivative thereof. It is considered that the solid dispersion obtained by the production method of the present invention exhibits the high stability of the rapamycin or a derivative thereof as a result of the interaction of the water-soluble polymeric carrier with the rapamycin or a derivative thereof. Hence, if the sugars whose additive amount is likely to be higher than that of other additives are present in the mixture during the interaction of the water-soluble polymeric carrier with the rapamycin or a derivative thereof, the sugars might inhibit the interaction of the water-soluble polymeric carrier with the rapamycin or a derivative thereof. Accordingly, the sugars are preferably added, after the water-soluble polymeric carrier has been mixed with the rapamycin or a derivative thereof.

A specific example of the production method of the present invention may preferably be a method for producing a solid dispersion, comprising:
a step of mixing a mixed solution prepared by mixing the water-soluble polymeric carrier (hypromellose, etc.) into the first solvent (ethanol, etc.) and/or the second solvent (acetone, etc.), a solution prepared by dissolving the stabilizer (tocopherol, etc.) in the first solvent (ethanol, etc.) and/or the second solvent (acetone, etc.), and a solution prepared by dissolving the rapamycin or a derivative thereof in the first solvent (ethanol, etc.) and/or the second solvent (acetone, etc.) with one another (wherein the ratio of the total amount of the used first solvents is higher than 50% by volume of the total volume of the used first solvents and second solvents);
a step of adding sugars (lactose hydrate, etc.) to the above-obtained mixed solution; and
a step of drying the above-obtained mixed solution to remove the solvents.

The solid dispersion produced by the production method of the present invention may comprise other additives commonly used in production of pharmaceutical preparations, to such an extent that such additives do not impair the effects of the present invention. Such other additives may be added in the first step.

When such other additives are used, the additives are preferably used in an amount of 0.01 to 10 parts by mass with respect to 1 part by mass of the rapamycin or a derivative thereof.

In addition, other additives may be added to a mixed solvent of the first solvent and the second solvent in the first step, and the thus added additives may be completely dissolved in the mixed solvent, or may also be suspended in the mixed solvent. Thus, other additives may be used in any aspects.

An excipient, a disintegrator, a binder, a lubricant, a pH adjuster, inorganic salts, and the like may be applied herein as other additives.

Examples of the excipient include sugars such as sucrose, erythritol, sorbitol, fucose, xylitol, fructose, inositol, and starch.

Examples of the disintegrator include carmellose, crospovidone, low-substituted hydroxypropyl cellulose, sodium starch glycolate, carmellose calcium, and croscarmellose sodium.

Examples of the binder include hydroxypropyl cellulose, hypromellose, polyvinyl alcohol, and polyvinyl pyrrolidone.

Examples of the lubricant include magnesium stearate, calcium stearate, sodium stearyl fumarate, talc, and sucrose fatty acid ester.

Examples of the pH adjuster include hydrochloric acid, sulfuric acid, phosphoric acid, citric acid, tartaric acid, malic acid, mesylic acid, tosylic acid, and besylic acid. A buffer, which comprises, as a main ingredient, such an acidic additive, and further, an alkaline metal salt, an alkaline-earth metal salt, or an ammonium salt, may also be used.

Examples of the inorganic salts include calcium chloride, sodium chloride, calcium oxide, and magnesium sulfate.

These additives can be used without any particular limitation, as long as they have purity that is acceptable for the intended use as pharmaceutical preparations. These additives may be used alone, or may also be used as a mixture of the additives. Such other additives are optionally used, when the solid dispersion is prepared.

Using the solid dispersion of the present invention which is prepared by the aforementioned method, a pharmaceutical preparation which comprises the solid dispersion can be prepared. Namely, a pharmaceutical preparation can be prepared by mixing the solid dispersion of the present invention with any additive which are commonly used in preparation of pharmaceutical preparation. Such other additives may comprise, for example, an excipient, a disintegrator, a binder, a lubricant, a pH adjuster, inorganic salts, and a solvent.

Examples of the excipient include sugars such as lactose, maltose, mannitol, sucrose, erythritol, sorbitol, fucose, xylitol, fructose, inositol, and starch.

Examples of the disintegrator include carmellose, crospovidone, low-substituted hydroxypropyl cellulose, sodium starch glycolate, carmellose calcium, and croscarmellose sodium.

Examples of the binder include hydroxypropyl cellulose, hypromellose, polyvinyl alcohol, and polyvinyl pyrrolidone.

Examples of the lubricant include magnesium stearate, calcium stearate, sodium stearyl fumarate, talc, and sucrose fatty acid ester.

Examples of the pH adjuster include hydrochloric acid, sulfuric acid, phosphoric acid, citric acid, tartaric acid, malic acid, mesylic acid, tosylic acid, and besylic acid. A buffer, which comprises, as a main ingredient, such an acidic additive, and further, an alkaline metal salt, an alkaline-earth metal salt, or an ammonium salt, may also be used.

Examples of the inorganic salts include calcium chloride, sodium chloride, calcium oxide, and magnesium sulfate.

In general, examples of the solvent include water, a normal saline, a 5% glucose or mannitol aqueous solution, a water-soluble organic solvent (e.g., a single solvent such as glycerol, ethanol, dimethyl sulfoxide, N-methylpyrrolidone, polyethylene glycol, Cremophor, or a mixed solvent thereof), and polyethylene glycols (e.g., polyethylene glycol 300, polyethylene glycol 400, polyethylene glycol 600, polyethylene glycol 4000, etc.).

These additives can be used without any particular limitation, as long as they have purity that is acceptable for the intended use as pharmaceutical preparation. These additives may be used alone, or may also be used as a mixture of the additives. Such other additives are optionally used, when the pharmaceutical preparation.

Examples of the dosage form of this pharmaceutical preparation include: internal use agents, such as a tablet, a dispersible tablet, a chewable tablet, an effervescent tablet, a troche, a drop agent, a hard capsule, a soft capsule, a granule, a powder agent, a pill, dry syrup, infusions and/or decoctions, an electuary, syrup, a drink agent, a suspension, an orally disintegrating tablet, and a jelly agent; and external use agents, such as a suppository, a poultice, a plaster, an ointment, a cream agent, a mousse agent solution, a liquid agent, eye drops, an aerosol agent, and a spray agent. The dosage forms are not limited thereto, but these are applicable preferred dosage forms.

In a case where the solid dispersion of the present invention is used in the form of an injection, examples of such an injection include an aqueous injection, a non-aqueous injection, a suspension injection, an emulsion injection, and also, as dosage forms of being dissolved or suspended at the time of use, an intradermal injection, a subcutaneous injection, an intramuscular injection, an intravenous injection, a central intravenous injection, an intra-arterial injection, and an intrathecal injection. Examples of the injection used herein are not limited thereto, but these injections can be used as applicable preferred dosage forms and administration routes.

The pharmaceutical preparation, in which the solid dispersion of the present invention is used, can be applied to the treatment of disease. Examples of the disease, to which the pharmaceutical preparation can be applied include: transplant rejection in transplantation of heart, lung, combined heart-lung, liver, kidney, pancreas, skin, or cornea; autoimmune diseases and inflammatory diseases, such as arthritis, rheumatic disease, systemic lupus erythematosus, multi polychondritis, crusts disease, Wegener's granulomatosis, dermatomyositis, chronic active hepatitis, myasthenia gravis, psoriasis, Steven-Johnson syndrome, idiopathic sprue, autoimmune inflammatory bowel disease, endocrine ophthalmopathy, Graves disease, nodule colitis, multiple sclerosis, primary biliary hepatitis, juvenile diabetes (type 1 diabetes), uveitis, keratoconjunctivitis sicca, vernal keratoconjunctivitis, interstitial lung fibrosis, psoriatic arthritis, glomerulonephritis, and juvenile dermatomyositis; asthma; and cancers and hyperproliferative diseases, such as breast cancer, renal cancer, neuroendocrine tumor, lymphoid proliferative disease, B cell lymphatic cancer, tuberous sclerosis, and proliferative skin disease. Examples of the disease are not limited thereto, but these diseases can be considered to be applicable preferred diseases.

The applied dose of the pharmaceutical preparation comprising the solid dispersion of the present invention can be naturally changed, depending on the sex, age, physiological conditions, pathologic conditions of a patient, etc. For example, the rapamycin or a derivative thereof is administered to an adult patient at a daily dose of 0.01 to 100 mg/m² (body surface area). The dose of the pharmaceutical product is not limited thereto, but this dose can be used as an applicable preferred dose.

### Examples

Hereinafter, the present invention will be more specifically described in the following examples. However, these examples are not intended to limit the scope of the present invention.

It is to be noted that, in the analysis using liquid chromatography (HPLC) in the present test examples, the measurement was carried out under the following conditions.

Measurement column: Zorbax Eclipse XDB-C18, Rapid resolution HT, 100 mm x 4.6 mm, 1.8 µm
Detector: ultraviolet absorption spectrophotometer (measurement wavelength: 278 nm)
Column temperature: 45°C
Mobile phase A: 0.1% formic acid; mobile phase B: methanol/acetonitrile = 50/50

### Concentration gradient of mobile phase:

**[Table 1]**

| Time after injectio (min) | Mobile phase A (vol %) | Mobile phase B (vol %) |
|---|---|---|
| 0 - 5 | 46 | 64 |
| 5 - 17 | 46 → 25 | 64 → 75 |
| 17 - 22 | 25 → 10 | 75 → 90 |
| 22 - 24 | 10 | 90 |
| 24 - 25 | 10 → 46 | 90 → 64 |
| 25 - 28 | 46 | 64 |

Flow rate: 1.5 mL/min
Injection amount: 10 µL
Peak analysis start time: 5 minutes
Peak analysis end time: 21.5 minutes

### [Example 1]

Into an Erlenmeyer flask, 500 mg of hypromellose (TC-5 E Type, manufactured by Shin-Etsu Chemical Co., Ltd.) was weighed, and thereafter, 14 mL of a mixed solution of ethanol and acetone (= 9 : 1) was added thereto, followed by stirring. To this mixed solution, 10 µL of an ethanol : acetone (= 9 : 1) solution containing 10 mg/mL tocopherol (manufactured by Riken Vitamin Co., Ltd.) and 10 µL of a heptane solution containing 100 mg/mL lecithin (manufactured by Junsei Chemical Co., Ltd.) were added, and thereafter, a solution prepared by dissolving 50 mg of everolimus in 500 µL of ethanol : acetone (= 9 : 1) was then added to the mixed solution, followed by stirring. Into this mixed solution, 50 mg of lactose hydrate (Pharmatose (registered trademark), manufactured by DFE Pharma) was added, and the obtained mixture was then stirred. The thus obtained mixed solution was spray-dried using a spray dryer (Mini Spray Dryer B-290, manufactured by BUCHI) to prepare a solid dispersion of Example 1.

### [Example 2]

Into an Erlenmeyer flask, 500 mg of hypromellose (TC-5 E Type, manufactured by Shin-Etsu Chemical Co., Ltd.) was weighed, and thereafter, 14 mL of a mixed solution of ethanol and acetone (= 7 : 3) was added thereto, followed by stirring. To this mixed solution, 10 µL of an ethanol : acetone (= 7 : 3) solution containing 10 mg/mL tocopherol (manufactured by Riken Vitamin Co., Ltd.) and 10 µL of a heptane solution containing 100 mg/mL lecithin (manufactured by Junsei Chemical Co., Ltd.) were added, and thereafter, a solution prepared by dissolving 50 mg of everolimus in 500 µL of ethanol : acetone (= 7 : 3) was then added to the mixed solution, followed by stirring. Into this mixed solution, 50 mg of lactose hydrate (Pharmatose (registered trademark), manufactured by DFE Pharma) was added, and the obtained mixture was then stirred. The thus obtained mixed solution was spray-dried using a spray dryer (Mini Spray Dryer B-290, manufactured by BUCHI) to prepare a solid dispersion of Example 2.

### [Comparative Example 1]

Into an Erlenmeyer flask, 500 mg of hypromellose (TC-5 E Type, manufactured by Shin-Etsu Chemical Co., Ltd.) was weighed, and thereafter, 14 mL of a mixed solution of ethanol and acetone (= 1 : 1) was added thereto, followed by stirring. To this mixed solution, 10 µL of an ethanol : acetone (= 1 : 1) solution containing 10 mg/mL tocopherol (manufactured by Riken Vitamin Co., Ltd.) and 10 µL of a heptane solution containing 100 mg/mL lecithin (manufactured by Junsei Chemical Co., Ltd.) were added, and thereafter, a solution prepared by dissolving 50 mg of everolimus in 500 µL of ethanol : acetone (= 1 : 1) was then added to the mixed solution, followed by stirring. Into this mixed solution, 50 mg of lactose hydrate (Pharmatose (registered trademark), manufactured by DFE Pharma) was added, and the obtained mixture was then stirred. The thus obtained mixed solution was spray-dried using a spray dryer (Mini Spray Dryer B-290, manufactured by BUCHI) to prepare a solid dispersion of Comparative Example 1.

### [Test Example 1]

Approximately 300 mg of the solid dispersion obtained in the method of each of Examples 1 and 2 and Comparative Example 1 was collected into a brown sample bottle without a lid, and was then stored in a desiccator in which the humidity was controlled at 40°C/saturated saline (relative humidity: approximately 75%) under light-shielding conditions.

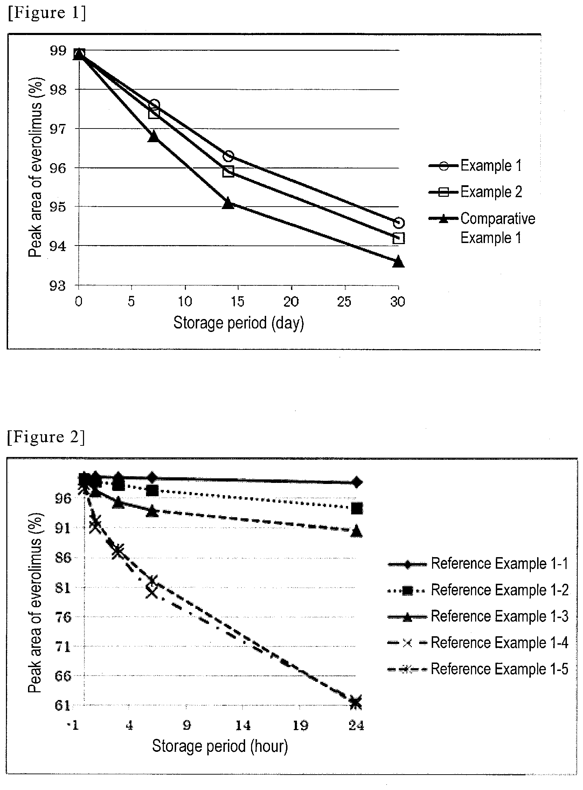

Seven days, 14 days, and 30 days after the storage test, the remaining amount of everolimus was measured by high performance liquid chromatography (HPLC), and the peak area ratio of everolimus at each time point was calculated. The results obtained by measuring the remaining ratio of everolimus are shown in Figure 1.

From the results shown in Figure 1, it became clear that the stability of the solid dispersion containing everolimus is largely changed depending on the type of the solvent used in preparation of the solid dispersion. In Comparative Example 1, the solid dispersion was prepared using the solvent disclosed in Patent Literature 1 (JP Patent Publication (Kohyo) No. 11-509223 A). The stability of this solid dispersion was poor under condition of 40°C/saturated saline (relative humidity: approximately 75%), and the purity of everolimus measured by HPLC was decreased by approximately 2%. On Day 14, the purity of everolimus was decreased by approximately 4%. On the other hand, it was demonstrated that, in the solid dispersions prepared in Example 1 and Example 2, in which the content of ethanol used as a solvent in preparation of the solid dispersions was increased, oxidation of everolimus, etc. is suppressed and high stability is exhibited, in comparison to known pharmaceutical compositions. These results demonstrate that the stability of everolimus in the solid dispersion is influenced not only by the composition of additives, but also by the solvents used in preparation of the solid dispersion. That is to say, when the content of ethanol in a mixed solvent of ethanol/acetone used in preparation of the solid dispersion is higher than 50%, the improvement of stability can be achieved. In Examples 1 and 2 relating to the present invention, the content of an active ingredient based on the expression of medicinal effects is guaranteed, and also, the expression of side effects that may be caused by decomposed products can be prevented. Therefore, it was demonstrated that the present invention can provide a pharmaceutical preparation, in which the active ingredient thereof such as everolimus has high stability and further, high safety.

### [Reference Example 1]

The stability test of everolimus in various mixed solvents was carried out. Various mixed solvents were each added to everolimus, and the prepared solutions each containing 1 mg/mL everolimus were then stored at 25°C.

The remaining amount of everolimus immediately after initiation of the storage test, and also 1 hour, 3 hours, 6 hours and 24 hours after initiation of the storage test was measured by high performance liquid chromatography (HPLC), and the peak area ratio of everolimus at each time point was then calculated. The ratio of solvents used for each sample is shown in Table 2, and the results obtained by measuring the remaining ratio of everolimus are shown in Figure 2.

**[Table 2]**

| Reference Example 1-1 | Only acetone |
|---|---|
| Reference Example 1-2 | Ethanol : acetone = 1 : 9 |
| Reference Example 1-3 | Ethanol : acetone = 2 : 8 |
| Reference Example 1-4 | Ethanol : acetone = 9 : 1 |
| Reference Example 1-5 | Only ethanol |

From the results shown in Figure 2, it was demonstrated that when the ratio of ethanol is increased, the purity of everolimus is decreased.

### [Reference Example 2]

The stability test of everolimus comprising a water-soluble polymeric carrier in a mixed solution of ethanol and acetone (= 9 : 1) was carried out. 10 mL of the mixed solution of ethanol and acetone (= 9 : 1) was added to 150 mg of hypromellose (TC-5 E Type, manufactured by Shin-Etsu Chemical Co., Ltd.), and the obtained mixture was then stirred. To this mixture, 10 mg of everolimus was added, and the thus prepared mixture was then stored at 25°C.

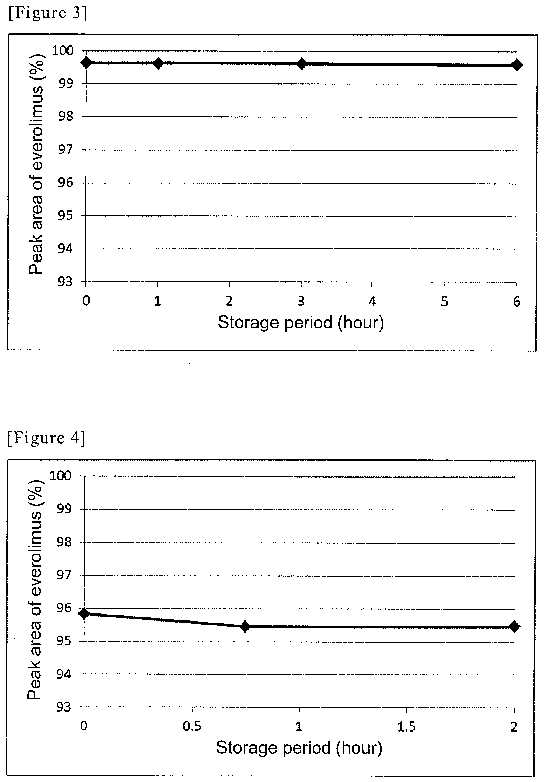

The remaining amount of everolimus immediately after initiation of the storage test, and also 1 hour, 3 hours and 6 hours after initiation of the storage test was measured by high performance liquid chromatography (HPLC), and the peak area ratio of everolimus at each time point was then calculated. The results obtained by measuring the remaining ratio of everolimus are shown in Figure 3.

From the results shown in Figure 3, it was demonstrated that the stability of everolimus is improved by the presence of hypromellose in a mixed solvent of ethanol and acetone (= 9 : 1).

### [Reference Example 3]

The stability test of everolimus comprising a water-soluble polymeric carrier in an ethanol solution was carried out. 10 mL of ethanol was added to 150 mg of hypromellose (TC-5 E Type, manufactured by Shin-Etsu Chemical Co., Ltd.), and the obtained mixture was then stirred. To this mixture, 10 mg of everolimus was added, followed by stirring, and thereafter, 15 mg of anhydrous lactose was added to the reaction mixture, and the obtained mixture was further stirred. The thus prepared mixture was stored at 25°C.

The remaining amount of everolimus immediately after initiation of the storage test, and also 0.75 hours and 2 hours after initiation of the storage test was measured by high performance liquid chromatography (HPLC), and the peak area ratio of everolimus at each time point was then calculated. The results obtained by measuring the remaining ratio of everolimus are shown in Figure 4.

From the results shown in Figure 4, it was demonstrated that the remaining ratio of everolimus was less than 96% in the ethanol solution, even immediately after the production thereof.

## Claims

1. A method for producing a solid dispersion comprising, as an active ingredient, rapamycin or a derivative thereof, comprising:
(1) a first step of mixing a first solvent that is a protic polar solvent, a second solvent that is an aprotic solvent, a water-soluble polymeric carrier, and rapamycin or a derivative thereof, wherein the ratio of the first solvent is higher than 50% by volume of the total volume of the first solvent and the second solvent; and
(2) a second step of removing the solvents.

2. The method for producing a solid dispersion according to claim 1, wherein the first solvent that is a protic polar solvent is one or more types selected from the group consisting of water, C1-C5 alcohol, and C1 and C2 carboxylic acid.

3. The method for producing a solid dispersion according to Claim 1 or 2, wherein the second solvent that is an aprotic solvent is one or more types selected from the group consisting of acetone, methyl ethyl ketone, methyl isobutyl ketone, anisole, methyl acetate, ethyl acetate, ethyl formate, propyl acetate, isopropyl acetate, n-butyl acetate, isobutyl acetate, acetonitrile, diethyl ether, t-butyl methyl ether, tetrahydrofuran, 1,4-dioxane, diisopropyl ether, pentane, hexane, heptane, N,N-dimethylformamide, N,N-dimethylacetamide, N-methyl pyrrolidone, 1,3-dimethyl-2-imidazolidinone, dimethyl sulfoxide, dichloromethane, and chloroform.

4. The method for producing a solid dispersion according to any one of Claims 1 to 3, wherein the volume of the first solvent is more than 2 parts by volume and less than 50 parts by volume, with respect to 1 part by volume of the second solvent.

5. The method for producing a solid dispersion according to any one of Claims 1 to 4, wherein the first solvent that is a protic polar solvent is ethanol, and the second solvent that is an aprotic solvent is acetone.

6. The method for producing a solid dispersion according to any one of Claims 1 to 5, wherein, in the first step, the first solvent is used in an amount of 15 parts by volume or more, with respect to 1 part by mass of the water-soluble polymeric carrier.

7. The method for producing a solid dispersion according to any one of Claims 1 to 6, wherein the water-soluble polymeric carrier is a water-soluble synthetic polymer derivative carrier or a water-soluble cellulose derivative carrier.

8. The method for producing a solid dispersion according to any one of Claims 1 to 7, wherein, in the first step, a mixed solution obtained by mixing the water-soluble polymeric carrier with the first solvent and/or the second solvent is mixed with a solution obtained by dissolving the rapamycin or a derivative thereof in the first solvent and/or the second solvent.

9. The method for producing a solid dispersion according to any one of Claims 1 to 8, wherein, in the first step, a stabilizer is added.

10. The method for producing a solid dispersion according to Claim 9, wherein the stabilizer is tocopherol.

11. The method for producing a solid dispersion according to Claim 9 or 10, wherein, in the first step, a mixed solution obtained by mixing the water-soluble polymeric carrier with the first solvent and/or the second solvent, a solution obtained by dissolving the stabilizer in the first solvent and/or the second solvent, and a solution obtained by dissolving the rapamycin or a derivative thereof in the first solvent and/or the second solvent are mixed with one another.

12. The method for producing a solid dispersion according to any one of Claims 1 to 11, wherein, in the first step, sugars are added.

13. A pharmaceutical preparation comprising a solid dispersion produced by the production method according to any one of Claims 1 to 12.
